# EUROPEAN PATENT APPLICATION

(11) **EP 4 599 765 A1**
(43) Date of publication of application: **13.08.2025**
(21) Application number: 24157162.9
(22) Date of filing: 12.02.2024
(51) Int. Cl.: A61B 5/11, A61B 5/16, A61B 5/00

(54) **ASSESSING SPATIAL COGNITION IN HUMAN SUBJECTS**

(71) Applicant: Silicon Neural Ltd., London EC1V 2NX (GB)
(72) Inventor: ROBERTS, Luke, London, EC1V 2NX (GB); DICKSON, Blair Thomas, London, EC1V 2NX (GB)
(74) Representative: Cross, James Peter Archibald

(57) **Abstract**

A method of assessing spatial cognition of a human subject comprises displaying to the subject on a two-dimension display screen, in at least one sequence of trials, a visual stimulus including a plurality of discrete visual orientation cues, the visual stimulus having a target location defined therein, wherein the rotational orientation of the visual stimulus is varied between trials within a sequence, and wherein the target location is hidden in a least some of the trials; receiving as input from the subject, in each said trial, an indication of the location of the target and recording data representing the displacement between the indicated location of the target and the defined target location in the at least one sequence of trials. There may be a plurality of sequences of trials, wherein an angular cue separation of the visual orientation cues varies between sequences, and the recording step includes recording displacement of the indicated location of the target from the defined target location for different values of said angular cue separation. The variation of displacement with angular cue separation may be analysed against an expected variation to provide an indication of spatial cognition in the subject.

## Description

### Technical Field

The present invention relates to a method, apparatus and computer program for assessing spatial cognition in human subjects.

### Background

Neurodegenerative disorders such as Alzheimer's disease (AD) result in a range of neuropsychological deficits that worsen over the course of the disease. In Alzheimer's disease deficits may include impairment in the ability to acquire and remember new information, impaired reasoning, impaired visuospatial abilities, impaired language functions and changes in behaviour whilst other neurodegenerative disorders present with similar or additional findings.

Neuroimaging, both structural and functional, has provided important evidence in the identification of the aetiology of changes in behaviour and the localisation of disease related changes in brain morphology. Notably some of the changes associated during the early stages of the disease affect the hippocampus, an area of the brain associated with the consolidation of both verbal and spatial memory.

Due to the overlapping nature of signs and symptoms associated with neurodegenerative disease, its diagnosis can be complex involving cognitive assessment, neuro-imaging and interviews with family members. Due to the complexity in diagnosis and the gradual onset of symptoms the mean time to diagnosis following symptoms onset can be prolonged.

Early identification of cognitive deficits using novel techniques may allow the earlier initiation of treatments that modify the course of disease in some neurodegenerative disorders. One approach to identify cognitive deficits in neurodegenerative disease is develop digital biomarkers. Digital approaches to data collection from patients are attractive due to the wide range of potential parameters for which data can be collected, the ability to collect data non-invasively and the opportunity to apply contemporary statistical techniques such as machine learning to derive novel insights from large data sets. New digital biomarker approaches also have the potential to be developed and applied as endpoints for the development of novel medicines without the need for complex or high cost assessments such as traditional cognitive assessment batteries, brain imaging or blood/cerebrospinal fluid biomarker.

### Statements of Invention

Aspects of the invention are defined by the accompanying claims.

Embodiments of the invention involve a non-interventional computer based task based on goal seeking behaviour. This may lead to improved understanding of human spatial cognitive processes in both normal and pathological states. Furthermore, assessment of specific behaviours related to goal seeking and spatial awareness may provide a sensitive mechanism with which to detect the presence and onset of disease associated with brain regions that are affected across a range of neuro-psychiatric disorders including but not limited to AD.

The computer-based task may involve displaying a two-dimensional stimulus, such as a circle, including visual orientation cues, such as differently coloured or shaded (e.g. white or black) arcs on the perimeter of a circle. The circle may rotate between individual tests or trials such that the position of the arcs on the perimeter changes. Within the circular stimulus is a target which is sometimes displayed (particularly in a training phase) and sometimes hidden. The position of the target rotates with the circle.

The subject indicates the expected position of the target when it is hidden, for example by touching the display screen or clicking at a position on the display screen with a mouse pointer. Between different tests or trials, the mutual separation of the cues may change.

The ability of the subject to locate the target, particularly when hidden, and optionally the dependency of this on the mutual separation of the cues, may be indicative of the spatial cognition of the subject. The differences between the indicated target locations and the defined positions of the target are recorded and input to a model which assesses spatial cognition of the subject.

The computer -based task may be performed by a computer program executed by a suitably configured computer system.

### Brief Description of Drawings

Specific embodiments will now be described with reference to the accompanying drawings as identified below.
Figures 1a and 1b show examples of visual stimuli used in an embodiment of the invention, with a defined target location respectively hidden and displayed.
Figure 2 is an outline flowchart of an experiment in an embodiment of the invention.
Figure 3 is a plot of defined target locations and user inputs of target locations in a first experiment.
Figure 4 is a version of Figure 3, normalised for rotation of visual stimuli in the experiment.
Figure 5 shows a vector field of the differences between normalised target locations and the respective user inputs for each trial in the first experiment.
Figure 6 is a chart of the average horizontal and vertical vector displacements for different angular separation of visual cues in the first experiment.
Figure 7 is a chart of net displacement based on the differences between the average horizontal and vertical vector displacements for different angular cue separations as shown in Figure 6.
Figure 8 is a graph showing predicted versus observed angular displacement vectors for different angular cue separation of visual cues in the first experiment.
Figure 9 is a graph showing linear regression of predicted versus observed angular displacement vectors across all observed values and angular cue separations.
Figure 10 is a graph showing linear regression for predicted versus observed radial displacement vectors across all observed values and angular cue separations in the first experiment.
Figure 11 is a plot of defined target locations and user inputs of target locations in a second experiment.
Figure 12 is a version of Figure 11, normalised for rotation of visual stimuli.
Figure 13 is a chart of the average horizontal and vertical vector displacements for different angular separation of visual cues in the second experiment.
Figure 14A is a graph showing angular target position plotted against angular goal displacement for different angular separation of visual cues in the second experiment.
Figure 14B is a graph showing radial target position plotted against radial goal displacement for different angular separation of visual cues in the second experiment.
Figure 15A is a graph showing the relationship between the predicted versus observed angular displacements in the second experiment.
Figure 15B is a graph showing the relationship between the predicted versus observed radial displacements in the second experiment.
Figure 16 is a set of plots of observed versus predicted angular and radial displacement for different values of cue separation in the second experiment.
Figure 17A is a plot of defined target locations and user inputs of target locations in a third experiment.
Figure 17B is a version of Figure 17A, normalised for rotation of visual stimuli.
Figure 18 is a chart of the average horizontal and vertical vector displacements for different angular separation of visual cues in the third experiment
Figure 19 is a set of plots of observed versus predicted angular and radial displacement for different values of cue separation in the third experiment.
Figure 20 is a flowchart representing, in outline, steps performed by a computer program for implementing an experimental sequence in an embodiment.
Figure 21 is a flowchart representing, in outline, data analysis steps performed in the data processing step of Figure 20.
Figure 22 is a schematic diagram of a computer system on which the computer program may be executed in an embodiment.

### Detailed Description of Embodiments

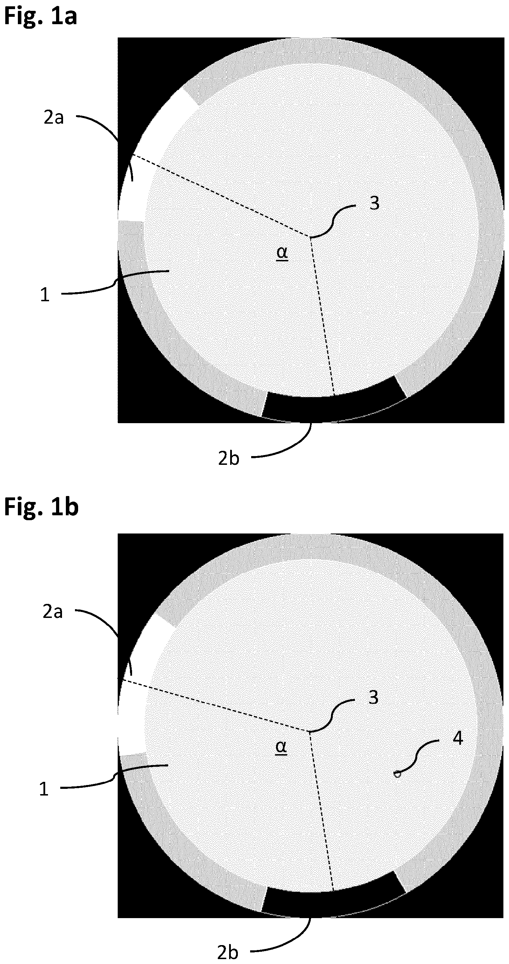

In an embodiment of the invention, computer software causes the presentation of visual stimuli on a computer screen. As shown in Figures 1a and 1b, the visual stimuli may comprise a circle 1 of constant diameter, having at least two cues 2a, 2b, in the form of contrasting annular arcs, for example a black arc and a white arc, with angular cue separation α as defined as the angle between lines (shown as dashed lines in Figures 1a and 1b) from the midpoints of the arcs to the centre 3 of the circle 1. Note that the dashed lines are shown to illustrate the angle α and are not displayed. A target 4 is defined at a position relative to the circle 1.

Each experiment comprises one or more sequences, each comprising a plurality of trials. In each sequence of trials, the target 4 is either hidden i.e. its position is defined by the computer software but not displayed as shown in Figure 1a, or is displayed as shown in Figure 1b.

The circle 1 together with the arcs 2a, 2b and the target 4 are rotated about the centre 3 between different trials within a sequence. The position of the target 4 relative to the circle 1 is constant between different trials, at least within a sequence, but rotates with the circle 1. This rotation may be perceived by the subject from the rotation of the cues 2a, 2b relative to the centre 3 of the circle 1.

In one example, the arcs 2a, 2b each subtend an angle of 45 degrees relative to the centre 3 and are separated by angle α of 135 degrees. The background (i.e. the rest of the area of the circle 1) may be displayed as a different colour or shade from the arcs 2a, 2b e.g. as a flat mid grey colour. The width of the annular arcs 2a, 2b was 95 pixels (2.5cm) and the diameter of the circle 1 was 568 pixels (about 15 cm). The target 4 is located within the circle 1 at least 75 pixels (2cm) from the perimeter of the circle 1 and at least 75 pixels from the centre 3 of the circle 1. The target 4 is circular, 20 pixels in diameter, and easily visible by the subject when displayed within the circle 1.

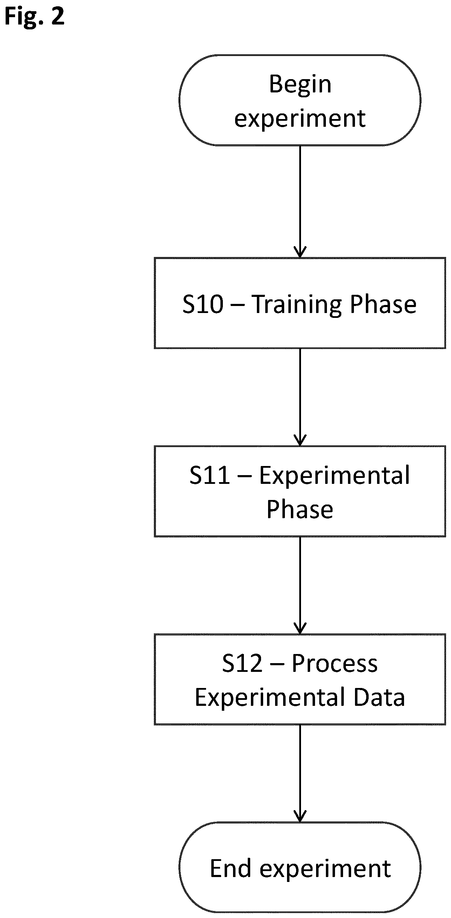

Figure 2 shows in outline the steps performed in an experiment to test cognitive spatial ability of a human subject.

At step S10, the subject participates in a training phase in which the software presents a first sequence of trials each with the target 4 visible, a second sequence of trials with the target 4 invisible, and a third sequence of trials with the target 4 visible. In each sequence of trials, the circle 1 is rotated by a random amount between each trial. In each trial, the circle 1 with arcs 2a, 2b is displayed on the screen, and the target 4 is displayed or hidden depending on the setting of the trial. The subject selects the location of the target 4 (e.g. by touching or pointing at the location on the display screen, or by clicking with a cursor such as a mouse pointer), the selected location is recorded, and the process proceeds to the next trial.

In one example, there were 15 training trials per subject with random rotation of the environment of between 1 and 360 degrees between each trial. The target 4 was visible in the first 5 and last 5 trials and invisible in the middle 5 trials.

In the training phase, the angular cue separation α between the arcs 2a, 2b is constant between each sequence of trials. For example, the angular cue separation α may be 135 degrees. Preferably, the position of the centre of the circle 1 is constant across all trials.

The training phase S10 may be used to familiarise the subject with the experiment and the data recorded during the training phase S10 need not be used for assessment of the subject.

In step S11, the subject participates in an experimental phase in which the software presents sequences of different trial types, each sequence comprising a plurality of trials of the same type. For example, the target may remain visible/invisible and the defined location of target 4 with respect to the circle 1 may remain fixed, but the circle 1 and cues 2a, 2b may rotate between different trials within the sequence. The angular cue separation α of the arcs 2a, 2b may vary between different sequences. In a subset of trial types, referred to as reinforcement trial types as in trial type 2 in Table 1 below, the target 4 is visible while in other trial types, it is hidden.

One example of the trials in the experimental phase is shown below in Table 1.

**Table 1 - Trial Types in Experimental Phase**

| **Sequence** | **No of trials** | **Target Visible** | **Cue separation** ° |
|---|---|---|---|
| 1 | 20 | N | 135 |
| 2 | 20 | Y | 135 |
| 3 | 5 | N | 147 |
| 4 | 5 | N | 159 |
| 5 | 5 | N | 123 |
| 6 | 5 | N | 111 |

The data output from the experimental phase includes the following parameters for each trial:
- Angle of cue separation e.g. relative to midpoint between the arcs 2a, 2b
- Angle of rotation of the circle 1 and of the arcs 2a, 2b
- Defined position of target 4 relative to the centre of the circle 1. This may be recorded in 2D Cartesian (x, y) coordinates and/or in polar (r, θ) coordinates.
- Position of target as selected by the subject, in same coordinates as position of target 4
Additional parameters may be recorded, such as the time taken to select the target position by the subject from the beginning of the display for each trial.

In one example, the display screen was a 21 inch (53 cm) diagonal monitor used in an indoor lighting (e.g. office) environment. Subjects were positioned approximately 50 cm from the computer screen. Instructions were provided to each subject verbally and a demonstration was given prior to the subject performing the training and experimental phases.

### Computer Program

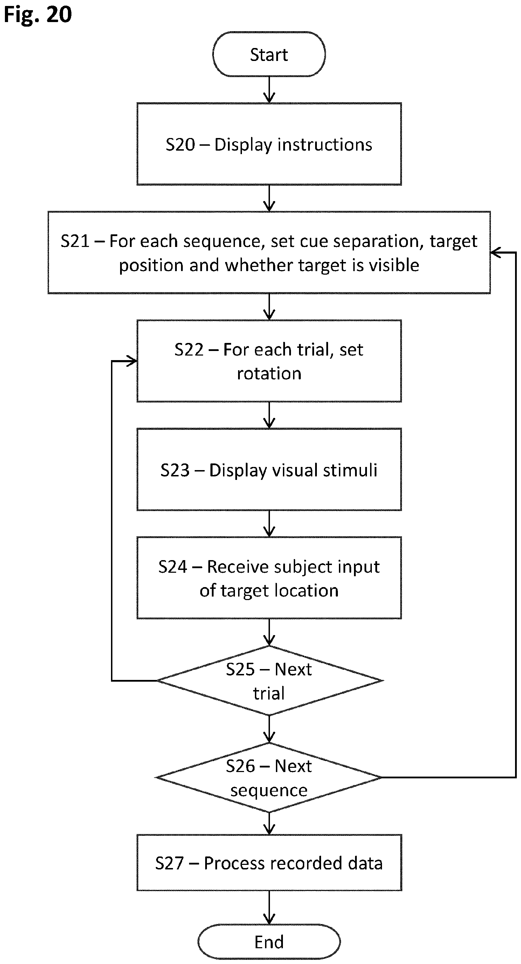

Figure 20 is a flowchart representing, in outline, steps performed by a computer program for implementing an experimental sequence such as outlined above.

First, at step S20, the program displays instructions to the subject for interacting with the computer during the experiment. Alternatively, the instructions may be provided separately, such as verbally or as printed instructions.

Next, at step S21, for each sequence of trials, the computer program determines the angular separation of visual cues for that sequence, the position of the target and whether the target 4 is visible. These settings may have been predetermined by an operator of the computer program and/or determined randomly or pseudo-randomly and recorded.

Next, at step S22, for each trial the rotation of the visual stimuli is determined as described above. The rotation for each trial may be selected randomly or pseudo-randomly, or may be predetermined.

In each trial, the visual stimuli are displayed to the subject (step S23), and an input of the selected target location is received by the subject (step S24). This step may time out if no input is received.

The computer program then proceeds to the next trial (step S25 returning to step S22). If the last trial in a sequence has been completed, the computer program proceeds to the next sequence (step S26 returning to step S21). If the last sequence in the experiment has been completed, the recorded data is processed as described above (step S27), although at least some data processing may be performed as the data is recorded and/or before the experiment has been completed. The process then ends.

The above sequence has been described in terms of 'for...next' loops, but the computer program is not dependent on any particular structure or logic.

Steps of the above process may be performed by different processors which may be remote from one another e.g. at different network nodes. For example, the input/output steps may be performed by a thin client local to the subject, while the control of the sequences and trials and the processing of recorded data may be performed by one or more remote processors.

The above process is described in terms of one or more sequences of one or more trials, with certain parameters such as target location and cue separation fixed within a sequence. Alternatively, the experiment may be structured in another way, for example by varying the cue separation within each sequence.

In any arrangement of trials, the subject should be shown the location of the target 4 before it is hidden, possibly with changes of rotation and/or cue separation, so that the subject can guess the location of the target 4. As in the example of Table 1 above, the location of the target 4 may be displayed to the subject during a training phase.

### Data Analysis

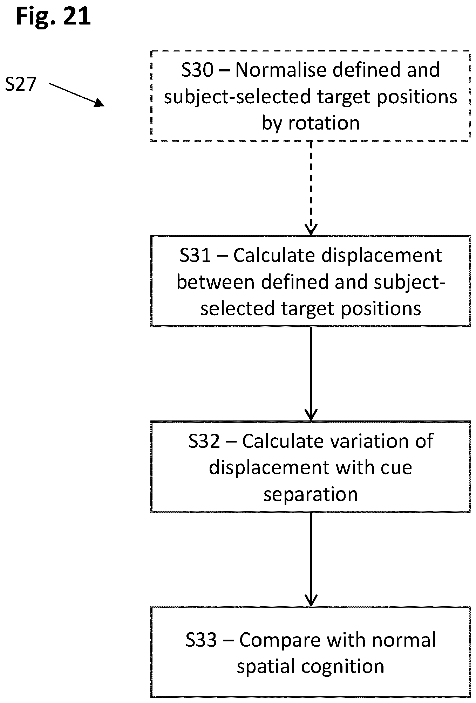

One example of data analysis in an embodiment of the invention is shown in in Figure 21.

The first task of the data analysis is to normalise (step S30) the defined and subject-selected positions of the target 4 for the angle of rotation of the visual stimuli e.g. the circle 1. This may be done by rotating the defined positions of the target 4 to a constant position and rotating the corresponding selected positions by the same rotation.

Next (step S31) the displacement (i.e. difference in position) between the defined and subject-selected positions of the target are calculated. The displacement may be calculated in Cartesian coordinates as a horizontal and a vertical displacement (DG.x, DG.y), and/or as a polar displacement (DG.r, DG.θ). In the case of calculating polar displacement, the normalisation step may be omitted because the rotation of the visual stimuli does not affect the polar displacement.

A mean of the displacement may be calculated across each trial in a sequence and/or for each value of cue separation. A variation of (e.g. a correlation between) the displacement (e.g. mean displacement) and the cue separation may then be calculated (step S32).

The variation between displacement and cue separation may be compared (step S33) with an expected variation characteristic of normal or abnormal spatial cognition, which may be determined empirically or may be based on a known model. For example, a correlation indicative of normal spatial cognition (e.g. based on experimental data or a model of human subjects having no impairment of spatial cognition) may be compared with the correlation for the candidate subject, and a difference between these correlations that is greater than the expected variation among normal subjects may be indicative of impaired spatial cognition. Alternatively or additionally, a correlation indicative of abnormal spatial cognition (e.g. based on experimental data or a model of human subjects having abnormal spatial cognition, for example associated with a specific condition) may be compared with the correlation for the candidate subject, and a difference between these correlations that is less than the expected variation among normal subjects may be indicative of abnormal spatial cognition.

Research on rats has demonstrated the behaviour of rat hippocampal place fields in response to changes in the external environment such as alterations in visual cues. A vector field equation has been proposed that predicts modifications of the representation of the environment in rat hippocampal place cells (see references below). A similar approach may be used to create a model of how the displacement is expected to vary with cue separation in human subjects.

### Experimental Results

A proof of concept study consisted of two separate experiments. In both experiments adult male and female subjects participated. In the first experiment a single adult subject performed the experimental sequence 10 times. In the second experiment 5 adult subjects (four males and one female) performed the experiment. No subject specific data other than sex was recorded for each of the subjects.

### Experiment 1

In the first experiment, a single subject conducted a single experimental sequence of the task. The purpose of this experiment was to obtain experience in the use of the software platform and provide initial data to support the analytical approach. Training sequences and trials with the target visible were removed from subsequent analysis. All further analysis is based on those parts of the experimental sequence in which the target is not visible.

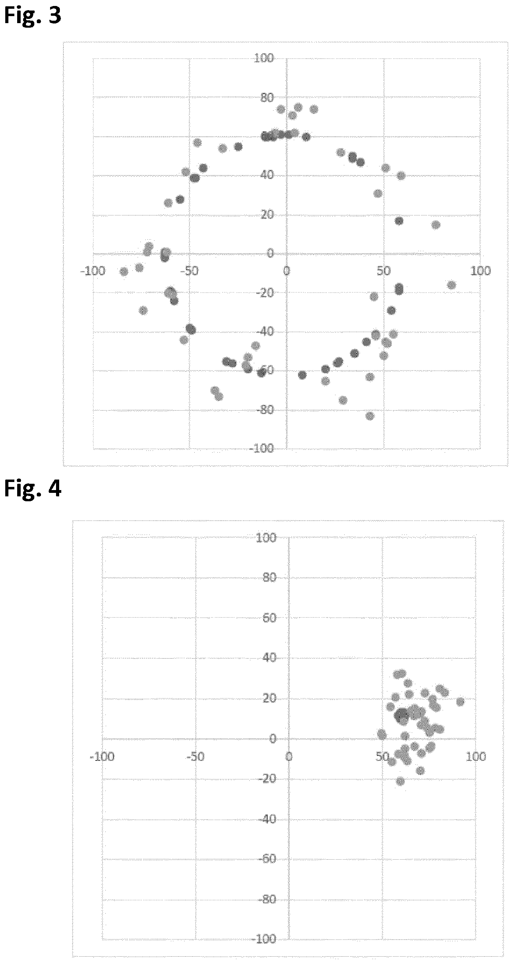

The results of the first experiment are shown in Figure 3, which shows the Cartesian coordinates of the defined target location in blue and the target location selected by the subject in orange. The results were then normalised by removing the effect of rotation to give the results shown in Figure 4. This shows a single defined target location since the defined location of the target 4 relative to the rotation of the circle 1 did not change during this experiment.

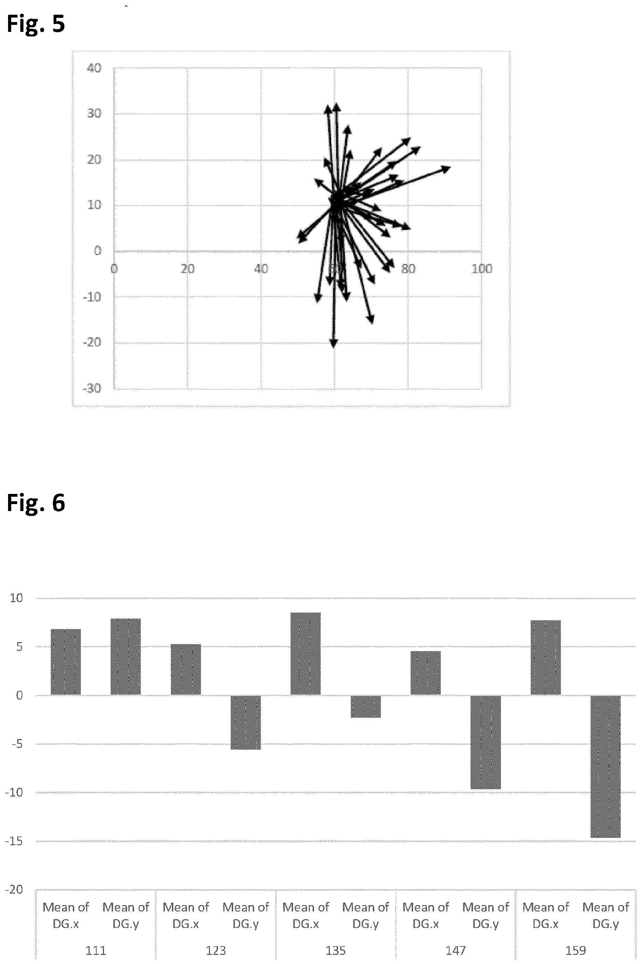

The results shown in Figure 4 may be plotted as a vector field as shown in Figure 5, i.e. with the displacement of each selected target location from the defined target location represented by a vector. The average horizontal and vertical displacement DG.x, DG.y of the vectors is then calculated for each value of cue separation, as shown for example in Figure 6. For this subject, the average magnitude of vertical displacement DG.y increases with increasing cue separation. The horizontal displacement DG.x appears generally consistent across all values of cue separation.

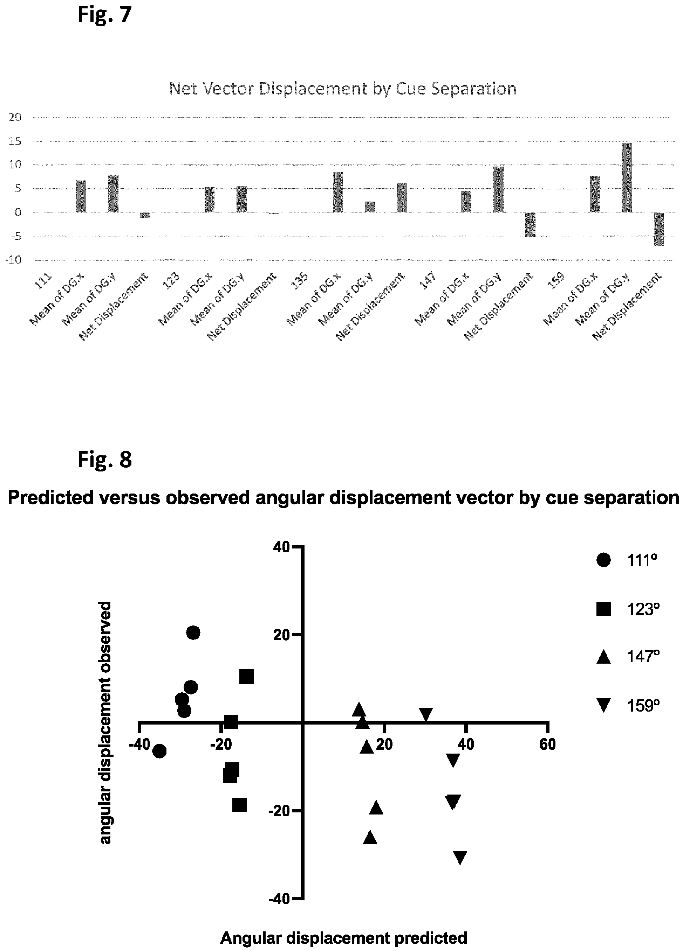

Net displacement can be expressed as the difference between the mean X and Y displacement vectors for each value of cue separation, as shown in Figure 7. The net displacement vectors were small at lower magnitude of cue separation and increase negatively as cue separation increases. The apparent progressive change in net displacement suggests that cue separation has an effect on target location selection and that the subject's selection of target location, when the target is not visible, is influenced by the location of the cues.

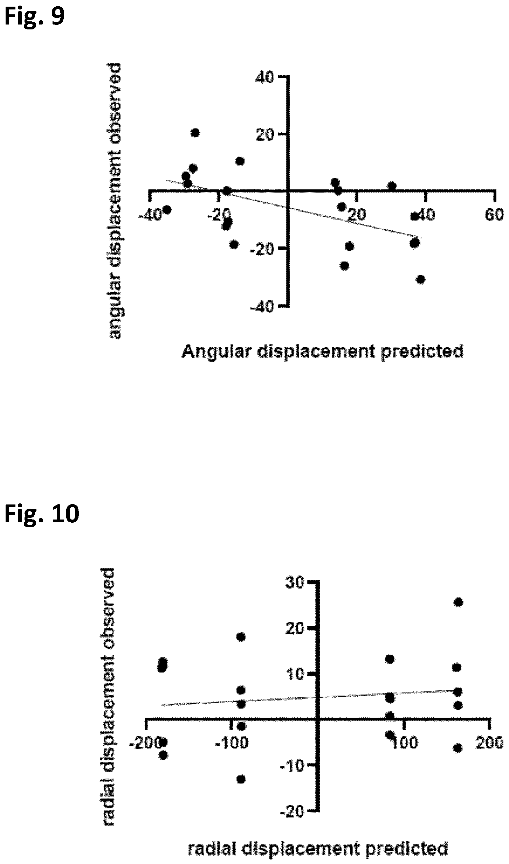

Figure 8 shows the locations predicted by the vector field equation versus observed locations across a sequence of five target locations for each value of cue separation. Considering all data points across all values of cue separation, not including the standard cue separation of 135 degrees, there is a significant correlation between predicted and observed values; see the linear regression shown in Figure 9 (R squared: 0.303, P=0.012) This suggests not only that the vector field equation may be effective in predicting certain elements of displacement, here angular displacement, but also that the subject is influenced by the location of the cues. Similarly to the angular displacement, the predicted radial displacement was plotted against the observed radial displacement as shown in Figure 10.

### Experiment 2

A second single subject experiment was performed in which a single subject performed multiple experimental sequences, with the target location being randomly selected and recorded for each sequence, and the circle 1 and cues 2a, 2b rotated together randomly between trials within a sequence. The purpose of this experiment was to increase the number of data points available for each value of cue separation. The subject performed a sequence of ten experimental sequences. Angular and radial displacement were calculated, as well as predicted and observed displacement.

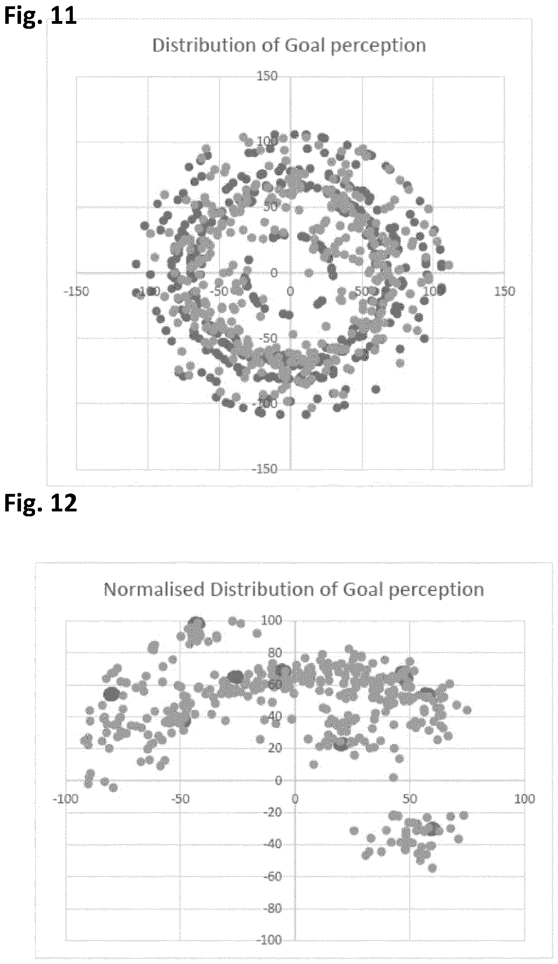

Figure 11 depicts the distribution of goal perception (i.e. selected target location) across the ten experimental sequences in the second experiment. The target location is depicted as concentric circles of blue dots consistent with the random presentation of the target being subsequently rotated. The orange dots represent the selected target location which is in less defined concentric circles. The locations were normalised for rotation between the trials, as shown in Figure 12. In this sequence, it is apparent that the random presentation resulted in most of the random locations of the target 4 appearing in the two upper quadrants with only one target location in the lower part of the circle 1; this location clearly shows the clustering of guess target locations around the defined target location.

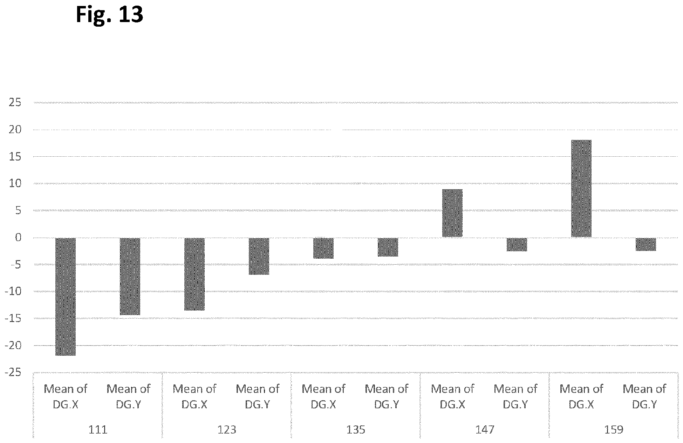

The mean horizontal and vertical displacement of the selected target location from the defined target location was calculated for each value of cue separation, as shown in Figure 13. Horizontal displacement increased as cue separation increased: at the smallest cue separation of 111 degrees horizontal displacement was most negative while at the widest separation the horizontal displacement was most positive. At the standard separation of 135 degrees the horizontal displacement was close to zero but slightly negative. In contrast to horizontal displacement, vertical displacement was most negative at the smallest separation but did not increase at wider cue separations beyond 135 degrees in this experiment.

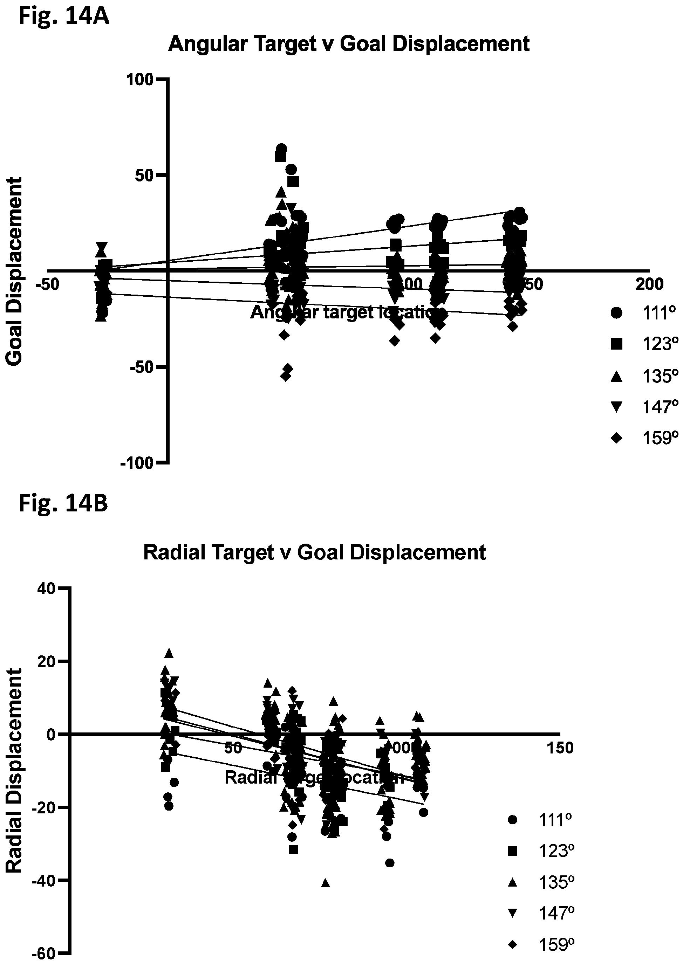

Angular target position was plotted against angular goal (i.e. subject-selected location) displacement as shown in Figure 14A, and radial target displacement against radial goal displacement as shown in Figure 14B. Considering all data points there was a significant effect of cue separation on angular target selection (Fig 14A) at 111, 123 and 159 degrees, as shown in Table 2 below. There was also significant negative correlation of the radial target with goal displacement across all values of cue separation (see Fig 14B and Table 3 below) with radial displacement decreasing as radial target location increased.

**Table 2: Linear regression of angular target versus goal displacement as a function of cue separation**

| **Cue separation** | **R²** | **P Value** |
|---|---|---|
| 111 | 0.3625 | <0.0001 |
| 123 | 0.1231 | 0.0125 |
| 135 | - | - |
| 147 | 0.0431 | 0.1477 |
| 159 | 0.0815 | 0.0445 |

**Table 3: Linear regression of radial target versus goal displacement as a function of cue separation**

| **Cue separation** | **R²** | **P Value** |
|---|---|---|
| 111 | 0.0306 | 0.2243 |
| 123 | 0.0793 | 0.0476 |
| 135 | - | - |
| 147 | 0.0019 | 0.7654 |
| 159 | 0.0057 | 0.0935 |

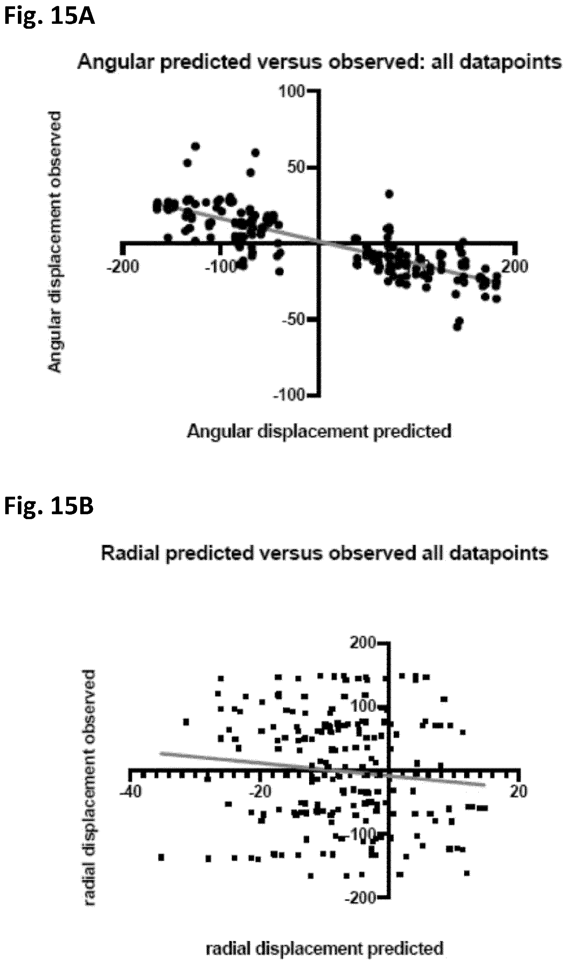

Figure 15 shows the relationship between the angular (Fig 15A) and radial (Fig 15B) predicted and observed displacements. The angular displacement demonstrates a clear negative correlation between the two values while the radial displacement fails to demonstrate a clear relationship with the individual values appearing in an apparently random distribution.

**Table 4 shows the predicted versus observed angular and radial displacements, confirming that the correlation between predicted and observed values of displacement were highly statistically significant for angular displacement but not for radial displacement.**

| **Displacement** | **R²** | **P Value** |
|---|---|---|
| Angular | 0.6567 | <0.0001 |
| Radial | 0.01180 | 0.1257 |

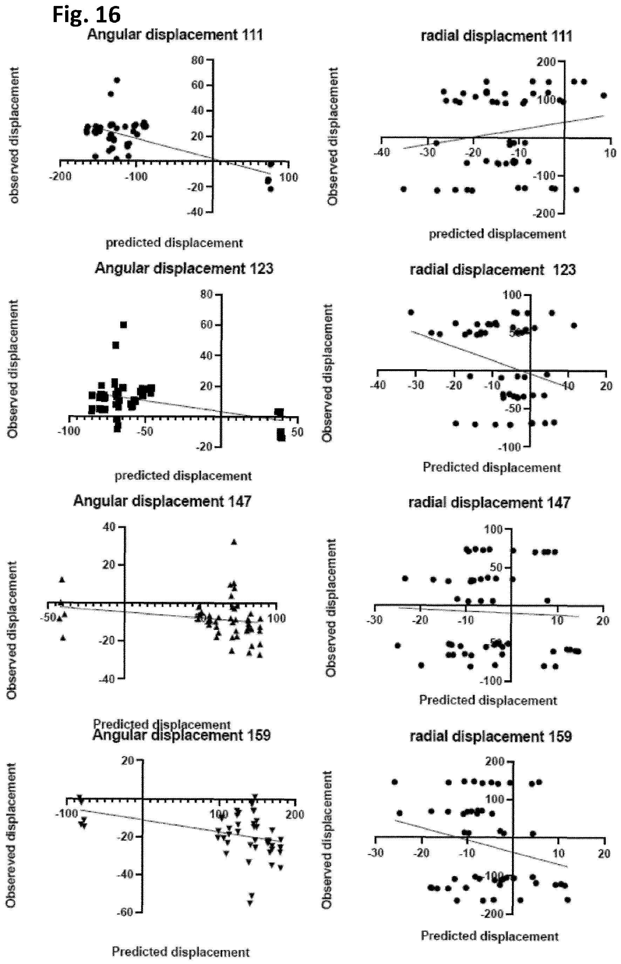

The effect of the individual cue conditions on observed and predicted displacement is shown in Figure 16 and Table 5 below. Significant correlation was observed across three of the four cue separation conditions with respect to angular displacement. With respect to radial displacement only one condition, cue separation at 123 degrees, displayed correlation between observed and predicted values.

**Table 5: predicted versus observed displacement as a function of cue separation.**

| **Displacement** | **R²** | **P value** |
|---|---|---|
| Angular 111 | 0.4532 | <0.0001* |
| Angular 123 | 0.1519 | 0.0051* |
| Angular 147 | 0.0415 | 0.1559 |
| Angular 159 | 0.1466 | 0.0061* |
| Radial 111 | 0.0306 | 0.2243 |
| Radial 123 | 0.0793 | 0.0476* |
| Radial 147 | 0.0019 | 0.7654 |
| Radial 159 | 0.0575 | 0.0935 |

### Experiment 3

In a third experiment, an initial cohort of 5 male and female subjects (4 male and 1 female) were recruited to test the experimental procedure and obtain X and Y co-ordinate data using a standard experimental sequence as described above. The tests were conducted on 21 inch monitors in an office environment, no attempt was made to standardise or minimise external cues i.e. desks, windows, lighting etc was visible and variable.

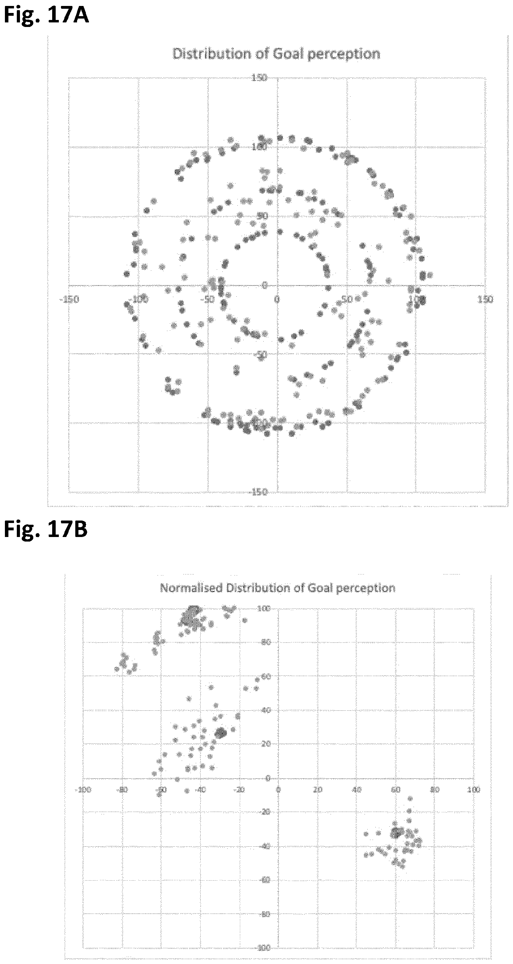

Using a small cohort of volunteers a limited number of targets were presented which limits interpretation but is sufficient to obtain a reasonable range of targets and subject target identification choices. The distribution of goal perception and the normalised distribution of goal perception is shown in Figures 17 A and B.

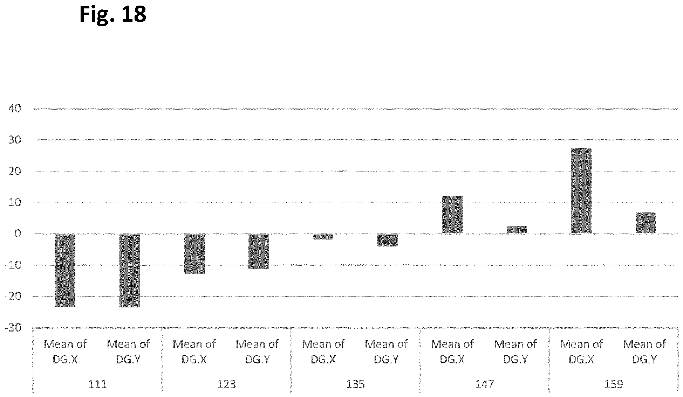

The mean horizontal and vertical displacement was determined across all subjects as a function of cue separation. Horizontal displacement increased with increasing separation above and below the standard cue separation being most negative at 111 degrees and most positive at 159 degrees. A similar pattern was observed with vertical displacement however displacement was greatest at minimum cue separation but attenuated with greater separation (see Fig. 18). Repeated measures analysis of variance (ANOVA) indicated a significant effect of cue separation (p<0.001) but not subject (p=0.71) on the variance.

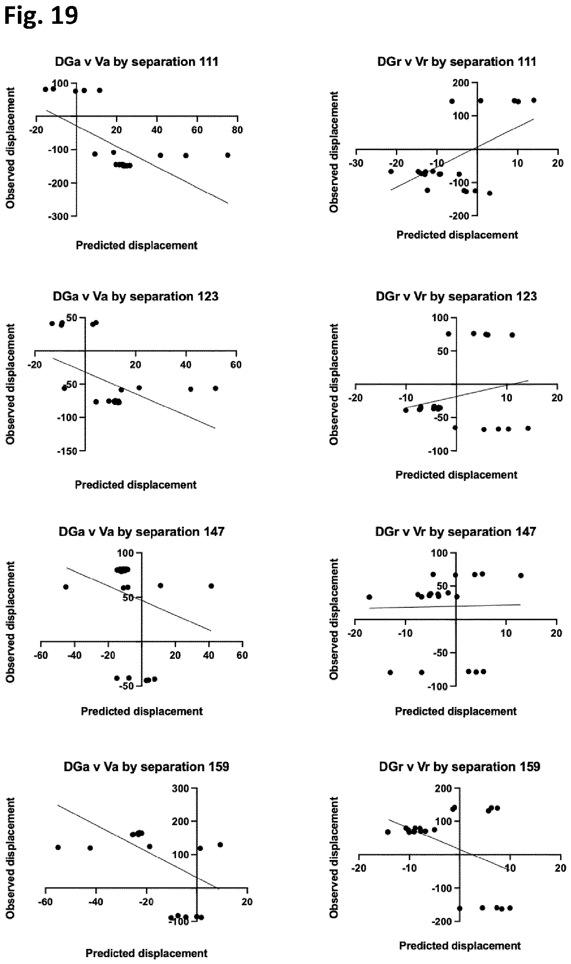

Predicted versus observed displacement of target selection in 5 subjects followed a similar pattern of behaviour to that seen in the single subject, multiple exposure test (referred to as Experiment 2) such that there was a negative relationship between the observed and predicted angular displacements (see Fig 19). Also similar to the single subject, multiple exposure test, the relationship between predicted and observed radial displacement values showed a more complex but consistent relationship with the relationship progressing from positive to negative with increasing separation of the cues.

In the case of angular displacement, a significant correlation between observed and predicted displacement was observed for cue separations of 111, 123 and 159 degrees but not 147 degrees. The correlation between the predicted and observed values appeared to be greater at smallest and largest cue separations.

With regards to radial displacement significant correlation between predicted and observed values was observed for cue separations of 111 and 159 degrees but not 123 or 147 degrees (see Table 6 below). However in the case of radial displacement the association between predicted and observed values was in opposing directions for the cue separations of 111 and 159 degrees which contrasts with angular displacement predictions which were directionally consistent across all cue separations.

**Table 6. Predicted versus observed angular and radial displacement as a function of cue separation**

| **Displacement** | **Cue Separation** | **R²** | **P value** |
|---|---|---|---|
| **Angular** | 111 | 0.3941 | 0.0008* |
| **Angular** | 123 | 0.2423 | 0.0124* |
| **Angular** | 147 | 0.0586 | 0.2437 |
| **Angular** | 159 | 0.2962 | 0.0049* |
| **Radial** | 111 | 0.3418 | 0.0021* |
| **Radial** | 123 | 0.0536 | 0.2655 |
| **Radial** | 147 | 0.0005 | 0.9175 |
| **Radial** | 159 | 0.2120 | 0.0206* |

### Summary of Experimental Results

Three experimental sequences were conducted involving 1. a single subject performing the one sequence, 2. a single subject performing ten sequences and 3. five subjects each performing one sequence.

In experiment 1 the impact of cue separation on horizontal or vertical displacement of target selection was unclear and this is likely due to the single data set. Nevertheless, net displacement showed an increasing trend with increasing cue separation especially at 147 and 159 degrees separation. There was a significant correlation of predicted and observed angular displacement but not radial displacement.

In experiment 2 a clear effect of cue separation on horizontal and vertical displacement was observed. This effect was particularly marked with horizontal displacement which demonstrated greater displacement at the widest and narrowest cue separations.

There was significant correlation between predicted and observed angular but not radial displacement overall. Considering the predicted and observed angular displacement for each cue separation condition, there was significant correlation in 3 of 4 cue conditions. There was a correlation between predicted and angular radial displacement in 1 of 4 cue conditions.

In experiment 3 there was again a clear effect of cue separation on horizontal and vertical displacement that mirrored that observed experiment 2.

The pattern of horizontal and vertical displacement in 2 subjects differed from the other three increasing the variability of these parameters in this group (data not shown).

Observed and predicted angular displacement was significantly correlated across 3 of 4 cue conditions while observed and predicted radial displacement was correlated in 2 of 4 cue conditions.

The experimental data shows that the experiment can be used to collect meaningful data on goal seeking behaviour. The performance of the vector field equation in predicting target displacement was improved with respect to angular compared to radial displacement. This suggests that an equation used to predict the behaviour of rat place cells also shows some ability to predict displacement of target selection in humans in an analogous task. This supports the hypothesis that similar neurophysiological processes underlie target selection both in rats and humans and may be related to place cell behaviour.

### Computer System

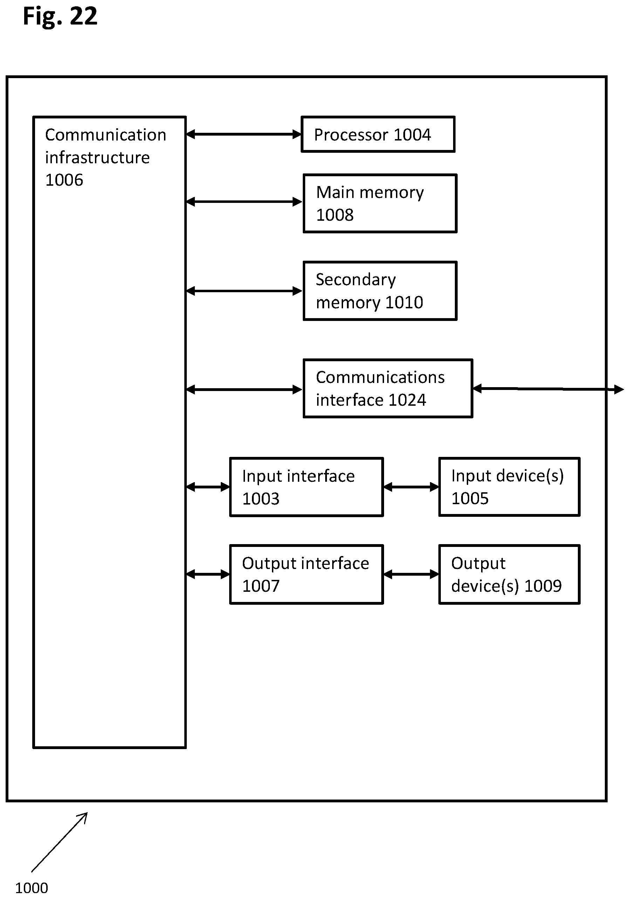

Figure 22 illustrates an example computer system 1000 in which the above computer program, or portions thereof, can be implemented as computer-readable code to program processing components of the computer system 1000. Various embodiments of the invention are described in terms of this example computer system 1000. For example, the computer-implemented experimental methods described above can be implemented in system 1000. After reading this description, it will become apparent to a person skilled in the relevant art how to implement the invention using other computer systems and/or computer architectures.

Computer system 1000 includes one or more processors, such as processor 1004. Processor 1004 can be a special purpose or a general-purpose processor. Processor 1004 is connected to a communication infrastructure 1006 (for example, a bus, or network). Computer system 1000 may include a user input interface 1003 connected to one or more input device(s) 1005 and an output interface 1007 connected to one or more output devices 1009, which may include a display for displaying the stimuli to the subject as described above. Input devices 1005 may include, for example, a connected device such as a mouse or touchpad, a keyboard, a touchscreen such as a resistive or capacitive touchscreen, etc.

Computer system 1000 also includes a main memory 1008, preferably random-access memory (RAM), and may also include a secondary memory 1010. Secondary memory 1010 may include, for example, a hard disk drive, a removable storage drive, flash memory, a memory stick, and/or any similar non-volatile storage mechanism. As will be appreciated by persons skilled in the relevant art(s), removable storage may include a non-transitory computer usable storage medium having stored therein computer software and/or data.

In alternative implementations, secondary memory 1010 may include other similar means for allowing computer programs or other instructions to be loaded into computer system 1000. Such means may include, for example, a removable storage unit and an interface which allow software and data to be transferred from the removable storage unit to computer system 1000.

Computer system 1000 may also include a communications interface 1024 implemented for example at the operating system level to allow data to be transferred between computer system 1000 and external devices, for example as signals over a communication channel. Communications interface 1024 may include a modem, a network interface (such as an Ethernet card), a communications port, a PCMCIA slot and card, or the like.

Various aspects of the present invention, such as the experimental methods described above, may be implemented by software and/or firmware (also called computer programs, instructions or computer control logic) to program programmable hardware, or hardware including special-purpose hardwired circuits such as application-specific integrated circuits (ASICs), programmable logic devices (PLDs), field-programmable gate arrays (FPGAs), etc. of the computer system 1000, or a combination thereof. Computer programs for use in implementing the techniques introduced here may be stored on a machine-readable storage medium and may be executed by one or more general-purpose or special-purpose programmable microprocessors. The terms "computer program medium", "non-transitory computer readable medium" and "computer usable medium" introduced herein can generally refer to media such as removable storage unit, removable storage unit, and a hard disk installed in hard disk drive. Computer program medium, computer readable storage medium, and computer usable medium can also refer to memories, such as main memory 1008 and secondary memory 1010, which can be memory semiconductors (e.g. DRAMs, etc.). These computer program products are means for providing software to computer system 1000.

Computer programs are stored in main memory 1008 and/or secondary memory 1010. Computer programs may also be received via communications interface 1024. Such computer programs, when executed, enable computer system 1000 to implement the present invention as described herein. In particular, the computer programs, when executed, enable processor 1004 to implement the processes of embodiments of the present invention as described above. Accordingly, such computer programs represent controllers of the computer system 1000. Where the invention is implemented using software, the software may be stored in a computer program product and loaded into computer system 1000 using removable storage or communications interface 1024.

### Alternative Embodiments

Alternative embodiments which may occur to the skilled person on reading the above description may nevertheless fall within the scope of the invention as defined by the following claims.

### References

1. Fenton AA, Csizmadia G, Muller RU. Conjoint control of hippocampal place cell firing by two visual stimuli. I. The effects of moving the stimuli on firing field positions. J Gen Physiol. 2000 Aug;116(2):191-209. doi: 10.1085/jgp.116.2.191. PMID: 10919866; PMCID: PMC2229496.
2. Fenton, G. Csizmadia, and R. U. Muller. Conjoint control of hippocampal place cell firing by two visual stimuli: II. a vector-field theory that predicts modifications of the representation of the environment. The Journal of general physiology, 116(2):211-222, 2000.

## Claims

1. A method of assessing spatial cognition of a human subject, comprising:
displaying to the subject on a two-dimension display screen, in a plurality of trials, visual stimuli including at least two rotational orientation cues having a mutual angular separation that varies between at least some of the trials, the visual stimuli having a target location defined therein, wherein a rotational orientation of the visual stimuli including the target location is varied between said trials, and wherein the target location is hidden in some of the trials;
receiving as input from the subject, in at least some of the trials, an indication of the location of the target; and
recording data representing displacement between the indicated location of the target and the defined target location at least when the target location is hidden, and corresponding values of said mutual angular separation of the rotational orientation cues.

2. The method of claim 1, wherein the displacement comprises a horizontal and/or vertical displacement in Cartesian coordinates, normalised for said rotational orientation of the visual stimuli.

3. The method of claim 1 or claim 2, wherein the displacement comprises a radial and/or angular displacement.

4. The method of any preceding claim, including analysing said recorded data so as to calculate a variation of said displacement as a function of said angular separation .

5. The method of claim 4, including comparing said variation with at least one variation representative of a condition affecting spatial cognition in a human subject.

6. The method of claim 4, including comparing said variation with at least one variation representative of normal spatial cognition in a human subject.

7. The method of any preceding claim, wherein the target location relative to the visual stimuli varies between at least some of the trials.

8. The method of any preceding claim, wherein the plurality of trials are arranged in one or more sequences each comprising a plurality of said trials.

9. The method of claim 8, wherein the target location relative to the visual stimuli is constant within the same sequence of trials.

10. The method of claim 8 or claim 9, wherein the mutual angular separation between the rotational orientation cues is constant within the same sequence of trials.

11. The method of any one of claims 8 to 10, wherein the target location either remains hidden or displayed within the same sequence of trials.

12. The method of any preceding claim, wherein the visual stimuli comprises a circle and the discrete visual orientation cues comprise discrete arcs of the circle.

13. A computer-readable storage medium comprising instructions which, when executed by a computer, cause the computer to carry out the method of any preceding claim.

14. Apparatus comprising a computer having a display screen and a user input device, the computer being configured to perform the method of any one of claims 1 to 12.
